**Europäisches Patentamt**

**European Patent Office**

**Office européen des brevets**

(11) Veröffentlichungsnummer: **0 006 203**
**B1**

(12)

# EUROPÄISCHE PATENTSCHRIFT

(45) Veröffentlichungstag der Patentschrift:
**16.09.81**

(51) Int. Cl.³: **C 07 C 97/24**

(21) Anmeldenummer: **79101862.5**

(22) Anmeldetag: **11.06.79**

(54) Verfahren zur Herstellung von Aminoanthrachinonen.

(30) Priorität: **21.06.78 DE 2827197**

(43) Veröffentlichungstag der Anmeldung:
**09.01.80 Patentblatt 80/1**

(45) Bekanntmachung des Hinweises auf die Patenterteilung:
**16.09.81 Patentblatt 81/37**

(84) Benannte Vertragsstaaten:
**CH DE FR GB**

(56) Entgegenhaltungen:
**DE-A-2 334 991**
**DE-A-2 409 542**
**DE-C-2 330 230**
**DE-A-2 526 651**
**CH-A-592 713**

(73) Patentinhaber: **BAYER AG, Zentralbereich Patente, Marken und Lizenzen, D-5090 Leverkusen 1, Bayerwerk (DE)**

(72) Erfinder: **Braden, Rudolf, Dr., Nothauser Feld 1, D-5068 Odenthal (DE)**
Erfinder: **Schroeder, Bernd, Dr., Im Wiesengrund 4, D-5068 Odenthal (DE)**
Erfinder: **Neeff, Rütger, Dr., Berta-von-Suttner-Strasse 22, D-5090 Leverkusen 1 (DE)**
Erfinder: **HORACEK, GTZ, DIPL.-ING., Bamberger Strasse 14, D-5090 Leverkusen 1 (DE)**
Erfinder: **THELEN, BERND, DIPL.-ING., Carl-Duisberg-Strasse 327, D-5090 Leverkusen 1 (DE)**

Verfahren zur Herstellung von Aminoanthrachinonen

Die vorliegende Erfindung betrifft ein neues Verfahren zur Herstellung von 1,5-Diamino-, 1,8-Diamino-, 1-Amino-6-nitro- und/oder 1-Amino-7-nitro-anthrachinen aus den entsprechenden Dinitroanthrachinonen durch Umsetzung mit Ammoniak bei erhöhter Temperatur in einem organischen Reaktionsmedium.

In der DE-PS 23 30 230 und der CH-PS 592 713 ist die Herstellung von 1,5- und/oder 1,8-Diaminoanthrachinon, in der DE-OS 23 34 991 die Herstellung von α-Amino-β'-nitroanthrachinonen durch Umsetzung von 1,5- und/oder 1,8-Dinitroanthrachinon bzw. α,β'-Dinitroanthrachinonen mit Ammoniak bei erhöhtem Druck in Äthern, aliphatischen oder cycloaliphatischen Kohlenwasserstoffen oder gegebenenfalls substituierten aromatischen Kohlenwasserstoffen bei Temperaturen von 100–220°C beschrieben. In diesen Verfahren werden Ammoniak und Nitroanthrachinon in Molverhältnissen von mindestens 4:1 (bei der Herstellung der Diaminoanthrachinone) und von mindestens 3:1 (bei der Herstellung der Amino-nitro-anthrachinone) eingesetzt.

Bei der Ausführung der Umsetzungen in technischem Massstab treten jedoch Schwierigkeiten auf, da sich die freiwerdende Reaktionswärme bei grossen Ansätzen nicht mehr in der erforderlichen Geschwindigkeit abführen lässt und infolgedessen unerwünschte Temperatursteigerungen auftreten, die zu einer Minderung der Ausbeute und der Produktqualität führen.

Es wurde nun gefunden, dass sich die unerwünschten Überhitzungen bei der Umsetzung vermeiden lassen und dass man wesentlich reinere Produkte erhält, wenn man die Umsetzung der Nitroanthrachinone mit Ammoniak unter Einhaltung der nachstehend angegebenen Verfahrensbedingungen vornimmt. Die Erfindung betrifft daher ein Verfahren zur Herstellung von 1,5-Amino-, 1,8-Diamino-, 1-Amino-6-nitro- und/oder 1-Amino-7-nitroanthrachinon durch Umsetzung der entsprechenden Dinitroanthrachinone mit Ammoniak in einem organischen Reaktionsmedium bei Temperaturen im Bereich von 130 bis 200°C, wobei Ammoniak bezogen auf 1 Mol Dinitroanthrachinon in Mengen von 5 bis 30 Mol eingesetzt wird, das dadurch gekennzeichnet ist, dass man

a) als organisches Reaktionsmedium aliphatische, cycloaliphatische und/oder aromatische Kohlenwasserstoffe einsetzt, wobei die aromatischen Kohlenwasserstoffe alkylsubstituiert und/oder im Kern chloriert sein können;

b) Ammoniak und einen Teil des organischen Reaktionsmediums in einem Rührautoklaven vorlegt und auf eine Temperatur im Bereich von 130 bis 175°C erhitzt;

c) das Dinitroanthrachinon suspendiert im restlichen organischen Reaktionsmedium im Verlaufe von 30 bis 150 Minuten in die Vorlage eindosiert, wobei man oberhalb des Füllstandes des Rührautoklaven Wärme abzieht und so die Temperatur im organischen Reaktionsmedium im Bereich zwischen 140 und 175°C und den Druck im Bereich von 5 bis 120 bar hält;

d) nach Beendigung der Zugabe von Dinitroanthrachinon die Temperatur im organischen Reaktionsmedium für 15 bis 150 Minuten um mindestens 10°C höher als in der Stufe c) und im Bereich von 170 bis 200°C und den Druck im Bereich von 30 bis 200 bar einstellt;

e) das Reaktionsgemisch aus dem Rührautoklaven in eine im Bereich von 10 bis 30 bar betriebene erste Destillationseinheit einspeist, in der Stickstoff und Ammoniak über Kopf genommen werden und in der die Verweilzeit im Abtriebsteil und im Sumpf im Bereich von 1 bis 30 Minuten liegt;

f) das Sumpfprodukt aus Stufe e) in eine im Bereich von 0,5 bis 5 bar betriebene zweite Destillationseinheit einspeist, in der Reaktionswasser und ein solcher Teil des organischen Reaktionsmediums über Kopf genommen wird, dass ein Sumpfprodukt mit einem Feststoffanteil im Bereich von 10 bis 40 Gew.-% anfällt;

g) das Sumpfprodukt aus Stufe f) entweder trocknet oder auf eine Temperatur unter 120°C abkühlt, die festen Anteile aus dem abgekühlten Sumpfprodukt abtrennt und die abgetrennten festen Bestandteile trocknet.

In das erfindungsgemässe Verfahren kann 1,5-, 1,6-, 1,7- und/oder 1,8-Dinitroanthrachinon eingesetzt werden, wie es bei beliebigen Herstellungsverfahren anfällt. Selbstverständlich werden beim Einsatz relativ reiner Dinitroanthrachinone relativ reine Aminoanthrachinone erhalten. Es ist deshalb bevorzugt, in das erfindungsgemässe Verfahren Dinitroanthrachinone einzusetzen, die einen Reinheitsgrad von 97% und höher aufweisen. Aus 1,5- und 1,8-Dinitroanthrachinon entsteht beim erfindungsgemässen Verfahren 1,5- und 1,8-Diaminoanthrachinon, aus 1,6- und 1,7-Dinitroanthrachinon entsteht 1-Amino-6-nitro- und 1-Amino-7-nitroanthrachinon. Es können die einzelnen Dinitroanthrachinone eingesetzt werden, aber auch Gemische der genannten Dinitroanthrachinone, beispielsweise ein Gemisch aus 1,5- und 1,8-Dinitroanthrachinon.

Ammoniak kann in das erfindungsgemässe Verfahren in handelsüblicher Form eingesetzt werden. Bezogen auf 1 Mol in Stufe c) eindosiertes Dinitroanthrachinon werden in Stufe b) 5 bis 30 Mol Ammoniak vorgelegt. Vorzugsweise werden dabei 15 bis 25 Mol Ammoniak vorgelegt.

Als organisches Reaktionsmedium werden aliphatische, cycloaliphatische und/oder aromatische Kohlenwasserstoffe eingesetzt, wobei die aromatischen Kohlenwasserstoffe alkylsubstituiert und/oder im Kern chloriert sein können. Als organisches Reaktionsmedium kommen aus den genannten Verbindungsklassen insbesondere solche Verbindungen in Frage, die unter Normalbedingungen und den erfindungsgemässen Reaktionsbedingungen überwiegend flüssig und inert sind. Der Begriff «inert» bedeutet hierbei, dass bei Durchführung des erfindungsgemässen Verfahrens über 95%, vorzugsweise über 99% des jeweils eingesetzten organischen Reaktionsmediums unverändert wiedergewonnen werden können.

Geeignete aliphatische und cycloaliphatische Kohlenwasserstoffe sind beispielsweise n-Pentan, n-Hexan, n-Heptan, Cyclohexan, Methylcyclohexan, Cyclododecan, Decalin, Cycloheptan, Cyclopentan, n-Decan, 1,2-Dimethylcyclohexan, 1,3-Dimethylcyclohexan, 1,4-Dimethylcyclohexan, 2,2-Dimethylpentan, 2,3-Dimethylpentan, 2,4-Dimethylpentan, 3,3,-Dimethylpentan, Isopropylhexan, Methylcyclohexan, 2-Methylheptan, 3-Methylheptan, 4-Methylheptan, 2-Methylhexan, 3-Methylhexan, 2-Methyloctan, 3-Methyloctan, 4-Methyloctan, 2-Methyl-pentan, 3-Methylpentan, n-Octan, Pentaisobutan, Triäthylmethan, 2,2,3-Trimethylpentan, 2,2,4-Trimethylpentan, 2,3,3-Trimethylpentan.

Geeignete aromatische Kohlenwasserstoffe sind beispielsweise Benzol, Toluol, o-, m-, p-Xylol, Isopropylbenzol, Trimethylbenzol, Diäthylbenzol, Tetramethylbenzol, Di-iso-propylbenzol, Isododecylbenzol, Naphthalin, Methylnaphthalin, Diphenyl, Diphenylmethan, o-, m-, p-Cymol, Dibenzyl, Dihydronaphthalin, 2,2'-Dimethyldiphenyl, 2,3'-Dimethyldiphenyl, 2,4'-Dimethyldiphenyl, 3,3'-Dimethyldiphenyl, 1,2-Dimethyl-naphthalin, 1,4-Dimethyl-naphthalin, 1,6-Dimethyl-naphthalin, 1,7-Dimethyl-naphthalin, 1,1-Diphenyläthan, Hexamethylbenzol, Isoamylbenzol, Pentamethylbenzol, 1,3,4-Tetramethylbenzol, 1,2,3,5-Tetramethylbenzol, 1,2,7-Trimethylnaphthalin, 1,2,5-Trimethylnaphthalin, Chlorbenzol, o-, m-, p-Dichlorbenzol, Chlortoluol, Chlorxylol, Äthyl-chlorbenzol, Chlornaphthalin.

Bevorzugt werden in das erfindungsgemässe Verfahren Toluol, o-Xylol, m-Xylol, p-Xylol, Xylol-Gemische, Trimethylbenzol, Chlorbenzol, o-Dichlorbenzol, m-Dichlorbenzol, p-Dichlorbenzol, Dichlorbenzol-Gemische, o-Chlortoluol, m-Chlortoluol, p-Chlortoluol, Chlortoluol-Gemische und Chlorxylole eingesetzt.

Man kann in das erfindungsgemässe Verfahren eine der vorgenannten Einzelverbindungen oder Gemische der genannten Verbindungen einsetzen. Als Gemische kommen insbesondere solche in Frage, deren Komponenten nahe beieinander liegende Siedepunkte aufweisen. Man kann auch Gemische verwenden, die aus einer relativ niedrig und einer relativ hoch siedenden Komponente bestehen. In diesem Fall kann man in Stufe f) so arbeiten, dass zusammen mit dem Wasser im wesentlichen nur die niedrig siedende Komponente über Kopf genommen wird.

Besonders bevorzugt ist der Einsatz von einzelnen Xylolen oder Xylolgemischen und der Einsatz von Chlorbenzol.

Das erfindungsgemässe Verfahren kann im allgemeinen in befriedigender Weise durchgeführt werden, wenn bezogen auf ein Gewichtsteil Dinitroanthrachinon 3 oder mehr Gewichtsteile des organischen Reaktionsmediums eingesetzt werden. Vorzugsweise werden zwischen 5 und 15 Gewichtsteile des organischen Mediums, bezogen auf 1 Gewichtsteil Dinitroanthrachinon, eingesetzt.

In Stufe b) des erfindungsgemässen Verfahrens wird das gesamte einzusetzende Ammoniak und ein Teil des organischen Reaktionsmediums eingesetzt. Der Teil des organischen Mediums, der in Stufe b) eingegeben wird, ergibt sich daraus, dass in Stufe c) eine etwa 10 bis 50% Feststoff enthaltende Suspension von Dinitroanthrachinon eingegeben werden kann. Derjenige Teil der Gesamtmenge des organischen Reaktionsmediums, der nicht in Stufe c) in Form der Suspension eingegeben wird, wird in Stufe b) eingegeben. Im allgemeinen lässt man sich im Rührautoklaven während des Vorlegens von Ammoniak und organischem Reaktionsmedium den sich von selbst einstellenden Überdruck aufbauen. Nach Beendigung der Eingabe von Ammoniak und organischem Reaktionsmedium wird durch Erhitzen eine Temperatur im Bereich von 130 bis 175 °C eingestellt. Beispielsweise kann diese Temperatur im Bereich von 140 bis 170 °C liegen, vorzugsweise im Bereich von 145 bis 165 °C.

In Stufe c) des erfindungsgemässen Verfahrens wird Dinitroanthrachinon in Form einer Suspension in dem organischen Reaktionsmedium in den Rührautoklaven im Verlaufe von 30 und 150 Minuten eindosiert. Die Suspension kann etwa 10 bis 50 Gew.-% Feststoff enthalten. Vorzugsweise enthält sie 20 bis 45 Gew.-% Feststoff. Der Druck vor und während der Zugabe der Suspension liegt im Bereich von 5 bis 120 bar, vorzugsweise im Bereich von 30 bis 100 bar. Im allgemeinen ist es völlig ausreichend, mit der Zugabe der Suspension bei dem Druck zu beginnen, der sich nach Stufe b) von selbst einstellt. Es kann in besonderen Fällen vorteilhaft sein, vor der Zugabe der Suspension einen höheren Druck als den sich selbst einstellenden einzustellen, z.B. indem man Inertgas, z.B. Stickstoff, aufdrückt, oder den Füllstand im Rührautoklaven erhöht, z.B. durch Einpumpen zusätzlicher Mengen des organischen Reaktionsmediums. Während der Zugabe der Suspension kann der Druck im Rührautoklaven ansteigen, fallen oder konstant gehalten werden. Die Zugabe der Suspension erfolgt vorzugsweise im Verlauf von 45 bis 90 Minuten.

Während des Zudosierens der Suspension wird im Rührautoklaven Wärme frei. Es ist ein wesentliches Merkmal des erfindungsgemässen Verfahrens, dass die Temperatur des organischen Reaktionsmediums während der Zugabe der Suspension im Bereich zwischen 140 und 175 °C gehalten wird. Vorzugsweise wird die Temperatur hierbei im Bereich 145 bis 165 °C gehalten. Erfindungsgemäss erfolgt die Abführung eines wesentlichen Teils der freiwerdenden Wärme aus einem Raum oberhalb des Füllstandes des Autoklaven. Beispielsweise kann diese Wärme mittels eines Wärmeaustauschers abgeführt werden, der im Gasraum des Rührautoklaven angeordnet ist. Die Abführung dieser Wärme kann auch mittels eines Wärmeaustauschers erfolgen, der oberhalb des eigentlichen Nutzraumes des Rührautoklaven angeordnet ist. In diesem Fall sollte jedoch sichergestellt sein, dass der Gasraum, aus dem die Wärmeabfuhr erfolgt, in gutem Diffusionskontakt zum Gasraum innerhalb des eigentlichen Nutzraums des Rührautoklaven steht. Es ist möglichst zu vermeiden, dass der Wärmeaustauscher mit der im Rührautoklaven befindlichen flüssigen Phase in Kontakt kommt. Ein geringer Teil der freiwerdenden Wärme kann auch durch Abstrahlung abgeführt werden.

In Stufe d) des erfindungsgemässen Verfahrens wird die Temperatur in dem nach der Stufe c) vorliegenden Reaktionsgemisch für 15 bis 150 Minuten um mindestens 10 °C erhöht, wobei man eine Temperatur von 200 °C nicht überschreitet. Vorzugsweise wird die Temperatur in Stufe d) mindestens um 15 °C erhöht und eine Temperatur von 185 °C nicht überschritten. Der Druck kann in Stufe d) bis auf 200 bar, vorzugsweise bis auf 150 bar ansteigen. Es ist auch möglich, aus der Gasphase des Rührautoklaven während der Stufe d) über ein Entspannungsventil, das nach einem Wärmeaustauscher angeordnet ist, den freiwerdenden Stickstoff abzulassen, so dass der Druck nicht ansteigt. In Stufe d) ist der Druck im Bereich 30 bis 200 bar, vorzugsweise im Bereich 70 bis 150 bar einstellbar.

Die Gesamtzeit für die Durchführung der Stufen c) und d) beträgt im allgemeinen bis zu 240 Minuten, vorzugsweise zwischen 90 und 180 Minuten.

In Stufe e) des erfindungsgemässen Verfahrens wird aus dem Reaktionsgemisch, das nach Stufe d) vorliegt, Ammoniak und gebildeter Stickstoff entfernt. Hierzu wird das Reaktionsgemisch aus Stufe d) in eine erste Destillationseinheit eingespeist, die bei einem Druck im Bereich von 10 bis 30 bar betrieben wird, und in der Ammoniak und Stickstoff über Kopf genommen werden. Diese erste Destillationseinheit kann beispielsweise aus einem oder mehreren Rührbehältern, gegebenenfalls mit aufgesetzter Kolonne, einem leeren Rohr, einem Dünnschichtverdampfer oder einer Rektifizierkolonne bestehen. Die Entfernung des Ammoniaks erfolgt bevorzugt auf Restgehalte von weniger als 0,5 Gew.-% (bezogen auf das gesamte Sumpfprodukt). Die Sumpftemperatur in dieser ersten Destillationseinheit hängt im wesentlichen von der Art des organischen Reaktionsmediums, der Menge des Reaktionswassers und dem Druck ab.

Dabei werden relativ hohe Sumpftemperaturen eingestellt, wenn ein relativ hochsiedendes organisches Reaktionsmedium eingesetzt und bei relativ hohen Drucken gearbeitet wird oder wenig Reaktionswasser im Sumpf anfällt. Bevorzugt liegt die Sumpftemperatur bei 200 °C oder darüber. Die Verweilzeit im Abtriebsteil und im Sumpf beträgt 1 bis 30 Minuten, wobei bei Sumpftemperaturen von 200 °C oder darüber 2 bis 12 Minuten bevorzugt sind. Wenn die Sumpftemperatur nicht über 200 °C betragen kann, z.B. weil ein relativ niedrig siedendes organisches Reaktionsmedium verwendet wird, sind längere Verweilzeiten bevorzugt, beispielsweise 10 bis 40 Minuten.

In einer bevorzugten Ausführungsform des erfindungsgemässen Verfahrens werden das am Kopf der ersten Destillationseinheit abgezogene Ammoniak und der Stickstoff über einen Kondensator geführt, in dem das Ammoniak abgetrennt wird. Das so wiedergewonnene Ammoniak kann dann in die Stufe b) zurückgeführt werden. Der nach dem Kondensator verbleibende Stickstoffstrom wird vorzugsweise einer Abgaswäsche, die z.B. mit Wasser durchgeführt wird, zugeführt und kann dann ohne Belastung für die Umwelt in die Atmosphäre abgegeben werden.

In Stufe f) des erfindungsgemässen Verfahrens wird aus dem Sumpfprodukt der Stufe e) das Reaktionswasser und ein Teil des organischen Reaktionsmediums entfernt. Hierzu wird das Sumpfprodukt aus Stufe e) in eine zweite Destillationseinheit eingespeist, die bei einem Druck im Bereich von 0,5 bis 5 bar betrieben wird. Diese zweite Destillationseinheit kann beispielsweise aus einem Dünnschichtverdampfer oder einem Rührbehälter, sowie aus einer Rektifizierkolonne bestehen. Vorzugsweise wird diese zweite Destillationseinheit bei Normaldruck betrieben. Der Austrag des Sumpfproduktes aus der Stufe e) in die Stufe f) kann über ein Reduzierventil oder eine Entspannungsdüse erfolgen. Es ist auch möglich, den Austrag über eine Schleuse vorzunehmen. Das Reaktionswasser wird in Stufe f) vollständig oder nahezu vollständig über Kopf genommen. Gleichzeitig wird soviel des organischen Reaktionsmediums über Kopf genommen, dass ein Sumpfprodukt anfällt, dessen Feststoffgehalt im Bereich 10 bis 40 Gew.-% liegt. Vorzugsweise liegt der Feststoffgehalt im Bereich 12 bis 20 Gew.-%. Dies kann in bestimmten Fällen erreicht werden, indem man nur die infolge der Druckentspannung sich bildenden Dämpfe entweichen lässt. Es ist durch Wärmezufuhr oder Kühlung möglich, mehr oder weniger Dämpfe entweichen zu lassen und so die gewünschte Feststoffkonzentration im Sumpfprodukt einzustellen.

In einer bevorzugten Ausführungsform der Stufe f) werden die entweichenden Dämpfe kondensiert, in eine wässrige und eine organische Phase getrennt und die organische Phase in die Stufe b) und/oder c) zurückgeführt. In einer weiteren bevorzugten Ausführungsform des erfindungsgemässen Verfahrens wird die verbleibende wässrige Phase in eine dritte Destillationseinheit eingeleitet, in der die in der wässrigen Phase noch vorhandenen Anteile des organischen Reaktionsmedium zusammen mit Wasser über Kopf genommen und in die Phasentrennung zurückgeführt werden. Als Sumpfprodukt fällt in der dritten Destillationseinheit dann ein von Schadstoffen nicht belastetes Abwasser an. Die dritte Destillationseinheit kann beispielsweise aus einer Rektifizierkolonne mit Verstärkungs- und Abtriebsteil bestehen.

In der Stufe g) des erfindungsgemässen Verfahrens wird aus dem Sumpfprodukt der Stufe f) das Reaktionsprodukt gewonnen. Hierzu kann das Sumpfprodukt aus Stufe f) direkt in einen üblichen Trockner eingespeist werden, in dem die noch vorhandenen Anteile des organischen Reaktionsmediums vollständig oder nahezu vollständig entfernt werden. Der Trockner kann gegebenenfalls unter vermindertem Druck und bei erhöhter Temperatur betrieben werden. Die Reinheit des Reaktionsproduktes kann im allgemeinen gesteigert werden, wenn man das Sumpfprodukt zunächst abkühlt und dann die festen Anteile abtrennt, z.B. durch Filtration oder Zentrifugation und dann die abgetrennten festen Anteile in einen üblichen Trockner einbringt. Man kann hier so verfahren, dass man zunächst auf eine Temperatur im Bereich von unter 120°C, beispielsweise auf eine Temperatur im Bereich von 30 bis 120°C, vorzugsweise auf eine Temperatur im Bereich von 40 bis 75°C abkühlt. Bei der Abtrennung der festen Anteile können mit der Mutterlauge Nebenprodukte entfernt werden, die sich aus dem organischen Reaktionsmedium gebildet haben können. Ein nennenswerter Verlust an gewünschtem Produkt tritt durch die Abtrennung der festen Anteile im allgemeinen nicht auf, da die gewünschten Produkte in der Mutterlauge ziemlich schwer löslich sind. Die optimalen Bedingungen für die Abtrennung der festen Anteile (z.B. Temperatur, Feststoffgehalt des abgekühlten Sumpfproduktes) können gegebenenfalls durch einfache Vorversuche ermittelt werden. Für die Abtrennung der festen Bestandteile sind insbesondere weitgehend automatisierte Apparate geeignet, z.B. Drehtrommelfilter.

In einer bevorzugten Ausführungsform der Stufe g) des erfindungsgemässen Verfahrens werden die bei der Trocknung entweichenden Brüden, gegebenenfalls gemeinsam mit der bei der Abtrennung der festen Anteile anfallenden Mutterlauge einer vierten Destillationseinheit zugeführt, in der die in den Brüden und gegebenenfalls der Mutterlauge vorhandenen Anteile des organischen Reaktionsmediums über Kopf abgetrennt und in Stufe b) und/oder c) zurückgeführt werden. Diese vierte Destillationseinheit kann beispielsweise aus einer Rektifizierkolonne mit Verstärkungs- und Abtriebsteil bestehen und gegebenenfalls unter vermindertem Druck betrieben werden.

Das erfindungsgemässe Verfahren kann diskontinuierlich oder kontinuierlich durchgeführt werden. Bei diskontinuierlicher Arbeitsweise werden die angegebenen Stufen in der angegebenen Reihenfolge durchlaufen, wobei die bei den einzelnen Stufen als besondere Ausführungsform bezeichneten Massnahmen unterbleiben können. Vorzugsweise wird das erfindungsgemässe Verfahren jedoch kontinuierlich durchgeführt, wobei die bei den einzelnen Stufen angegebenen besonderen Ausführungsformen mit durchgeführt werden.

Bei kontinuierlicher Arbeitsweise werden die Stufen b), c) und d) in mehreren parallel geschalteten, zeitlich gegeneinander versetzt arbeitenden Rührautoklaven durchgeführt. Infolge der zeitlichen Versetzung verlassen die Reaktionsgemische die Reaktoren auch zeitlich versetzt und treten zeitlich versetzt, d.h. nacheinander in die erste Destillationseinheit (Stufe e) ein. D.h. durch die Anwendung mehrerer parallel geschalteter, zeitlich gegeneinander versetzt arbeitender Rührautoklaven wird ein insgesamt kontinuierlicher Zustrom in Stufe e) erreicht. Der aus Stufe e) austretende kontinuierliche Strom durchläuft die folgenden Stufen f) und g) ebenfalls kontinuierlich und das Endprodukt fällt kontinuierlich an. Die Anzahl der Rührautoklaven beträgt bei kontinuierlicher Durchführung des erfindungsgemässen Verfahrens mindestens 2, vorzugsweise 3 bis 5.

Das erfindungsgemässe Verfahren hat eine Reihe von Vorteilen, die nicht vorhersehbar waren. So können nach dem erfindungsgemässen Verfahren die angegebenen Diamino- und Amino-nitroanthrachinone in Reinheiten von 95% und mehr erhalten werden, wenn man von entsprechend reinen Dinitroanthrachinone ausgeht. Wenn in Stufe g) die Filtration durchgeführt wird, beträgt die Reinheit der Produkte im allgemeinen über 97%. Die Produkte sind im allgemeinen reiner als bei den bisher bekannten Verfahren und so rein, dass sie im allgemeinen ohne weitere Reinigungsverfahren zur Herstellung von Farbstoffen verwendet werden können.

Beim erfindungsgemässen Verfahren kann unter Einsatz von nicht besonders grossen Mengen Ammoniak in relativ kurzen Reaktionszeiten in technischem Massstab in kontinuierlicher Arbeitsweise und unter praktisch vollständiger Rückführung des organischen Reaktionsmediums und des überschüssigen Ammoniaks ein sehr reines Produkt erhalten werden. Dabei kann so verfahren werden, dass neben dem gewünschten Produkt nur geringe Mengen eines von Schadstoffen nicht belasteten Abwassers und praktisch reiner Stickstoff anfallen. Die Reaktion zwischen Ammoniak und Dinitroanthrachinonen kann bei Bedingungen begonnen werden, die eine relativ niedrige Anfangstemperatur zulassen. Die Abführung der Reaktionswärme, bei der Herstellung von 1,5-Diaminoanthrachinon sind es etwa $10^6$ J/Mol, ist ohne Überschreitung bestimmter Temperaturgrenzen möglich, was zur Folge hat, dass Produkte hoher Reinheit mit sehr guten Selektivitäten erhalten werden und keine plötzlichen, schwierig kontrollierbaren Druckanstiege auftreten. Zusätzliche Hilfsstoffe, z.B. zusätzliche Fällungsmittel zur Abtrennung der Produkte, brauchen nicht eingesetzt zu werden.

Die erfindungsgemäss herstellbaren Diamino- und Amino-nitroanthrachinone können in an sich bekannter Weise zur Herstellung von Farbstoffen weiterverwendet werden. (Siehe z.B. Colour Index 61725, DOS 1644607, DOS 2300544, DOS 2300592).

Die folgenden Beispiele erläutern das erfindungsgemässe Verfahren, ohne es darauf einzuschränken.

Beispiele

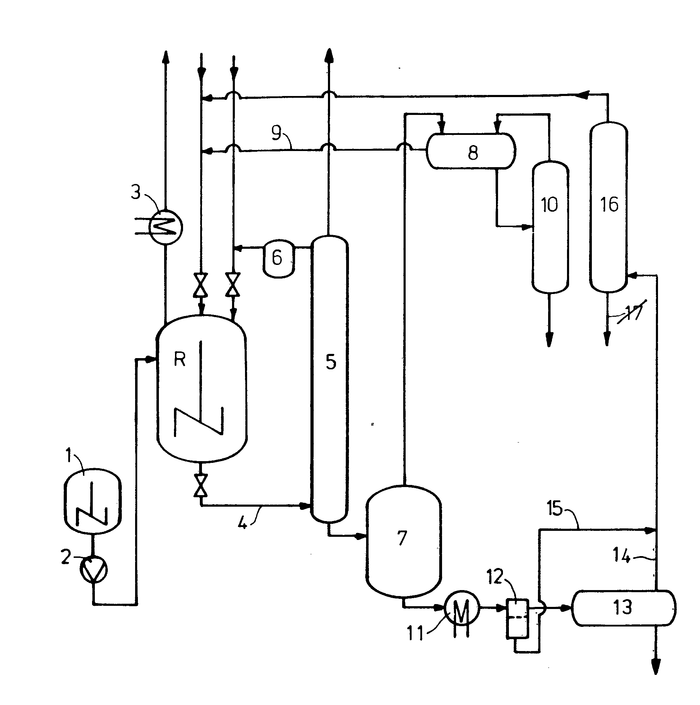

Beispiel 1 (Die Bezugszeichen beziehen sich auf die Fig. 1)

In einem von 2 parallel geschalteten Rührautoklaven (R), von denen in Fig. 1 nur einer abgebildet ist, bestehend aus rostfreiem Stahl, werden 110 kg Ammoniak und 1241 kg Dekalin eingepumpt und auf 160 °C erhitzt, so dass sich ein Druck von 45 bar einstellt. In einem Rührbehälter (1) werden 182,1 kg 1,5-Dinitroanthrachinon (Reinheit 98,8%) in 530 kg Dekalin suspendiert. Mit einer Kolbendosierpumpe (2) wird diese Suspension im Verlauf von 60 Minuten in den Rührautoklaven eindosiert. Die Temperatur im Autoklaven wird durch Wärmeabführung über einen Wärmeaustauscher (3), der über den Füllstand im Rührautoklaven und in einem Raum angebracht ist, der in gutem Diffusionskontakt mit dem Gasraum im Rührautoklaven steht, bei 160 bis 170 °C gehalten. Nach dem Zudosieren wird die Temperatur im Reaktor auf 195 °C erhöht und bei dieser Temperatur 15 Minuten gehalten.

Nach einer Reaktionszeit von insgesamt 115 Minuten ist das 1,5-Dinitroanthrachinon vollständig umgesetzt. Im Reaktor liegen 141,1 kg 1,5-Diaminoanthrachinon vor.

Die 2 parallel geschalteten Rührautoklaven werden zeitlich gegeneinander versetzt auf die gleiche Weise betrieben, und ein kontinuierlicher Strom des in Dekalin suspendierten 1,5-Diaminoanthrachinons über Leitung 4 in eine Ammoniak-Destillation (5) so eingebracht, dass je Stunde etwa 141 kg reines 1,5-Diaminoanthrachinon aus einem der Rührautoklaven ausgetragen werden. Bei einer Sumpftemperatur von 210 °C und 20 bar Druck werden Stickstoff und Ammoniak aus dem Reaktionsgemisch ausgetrieben. Ammoniak wird über einen Behälter (6) in kondensierter Form einem der Reaktoren (R) zugeführt. Stickstoff wird über eine Abgaswäsche mit Wasser in die Atmosphäre abgegeben.

Nach einer Verweilzeit von 36 Minuten im Sumpf der Ammoniak-Destillation (5) wird das Reaktionsgemisch auf Normaldruck in einen Behälter (7) entspannt. Dabei destilliert Wasser und Dekalin ab, die in einem Trenngefäss (8) nach Phasen getrennt werden. Dekalin wird über die Leitung (9) in einen der Rührautoklaven (R) zurückgeführt. Die wässrige Phase wird einer Destillation (10) zugeführt. Dort wird praktisch das ganze in der wässrigen Phase enthaltene Dekalin zusammen mit Wasser über Kopf genommen und in den Trennbehälter (8) zurückgeführt. Als Sumpfprodukt verbleibt ein schadstofffreies Abwasser.

Die Produktsuspension aus dem Behälter (7) wird über einen Kühler (11) mit 75 °C auf einen Filter (12) gebracht. Das Kristallisat wird einem Trockner (13) zugeführt und bei ca. 200 °C getrocknet. Die Brüden des Trockners (14) werden zusammen mit der Mutterlauge aus der Filtration (15) in eine Destillation (16) geführt. Dort wird Dekalin abdestilliert und in einen der Rührautoklaven (R) zurückgeführt. Aus dem Trockner werden stündlich 143 kg 1,5-Diaminoanthrachinon in einer Reinheit von 97,5% entnommen.

Beispiel 2 (Die Bezugszeichen beziehen sich auf die Fig. 1)

In einen von 4 parallel geschalteten Rührautoklaven (R), von denen in Fig. 1 nur einer abgebildet ist, bestehend aus rostfreiem Stahl, werden 310 kg Ammoniak und 1230 kg Chlorbenzol eingepumpt und auf 150 °C erhitzt. In einem Rührbehälter (1) werden 282,7 kg 1,5-Dinitroanthrachinon (Reinheit 98,8%) in 430 kg Chlorbenzol suspendiert. Mit einer Kolbendosierpumpe (2) wird diese Suspension im Verlauf von 60 Minuten in den Rührautoklaven eindosiert. Die Temperatur im Autoklaven wird durch Wärmeabführung über einen Wärmeaustauscher (3), der über dem Füllstand im Rührautoklaven und in einem Raum angebracht ist, der in gutem Diffusionskontakt mit dem Gasraum im Rührautoklaven steht, bei 150 bis 160 °C gehalten. Gegen Ende des Zudosierens ist der Druck im Autoklaven auf 100 bar angestiegen. Nach dem Zudosieren wird die Temperatur im Reaktor auf 180 °C erhöht und bei dieser Temperatur 45 Minuten gehalten.

Nach einer Reaktionszeit von insgesamt 150 Minuten ist das 1,5-Dinitroanthrachinon vollständig umgesetzt. Im Reaktor liegen 217,7 kg 1,5-Diaminoanthrachinon vor, was einer Selektivität von 97,6% entspricht.

Die 4 parallel geschalteten Rührautoklaven werden zeitlich gegeneinander versetzt auf die gleiche Weise betrieben, und ein kontinuierlicher Strom des in Chlorbenzol suspendierten 1,5-Diaminoanthrachinons über Leitung 4 in eine Ammoniak-Destillation (5) so eingebracht, dass je Stunde 145,1 kg reines 1,5-Diaminoanthrachinon aus einem der Rührautoklaven ausgetragen werden. Bei einer Sumpftemperatur von 200 °C und 20 bar Druck werden Stickstoff und Ammoniak aus dem Reaktionsgemisch ausgetrieben. Ammoniak wird über einen Behälter (6) in kondensierter Form einem der Reaktoren (R) zugeführt. Stickstoff wird über eine Abgaswäsche mit Wasser in die Atmosphäre abgegeben.

Nach einer Verweilzeit von 6 Minuten im Sumpf der Ammoniak-Destillation (5) wird das Reaktionsgemisch auf Normaldruck in einen Behälter (7) entspannt. Dabei destillieren stündlich 46 kg Wasser und 350 kg Chlorbenzol ab, die in einem Trenngefäss (8) nach Phasen getrennt werden. Chlorbenzol wird über die Leitung (9) in einen der Rührautoklaven (R) zurückgeführt. Die wässrige Phase wird einer Destillation (10) zugeführt. Dort wird praktisch das ganze in der wässrigen Phase enthaltene Chlorbenzol zusammen mit Wasser über Kopf genommen und in den Trennbehälter (8) zurückgeführt. Als Sumpfprodukt verbleibt ein schadstofffreies Abwasser.

Die Produktsuspension aus dem Behälter (7) wird über einen Kühler (11) mit 50 °C auf einen Filter (12) gebracht. Das Kristallisat wird einem Trockner (13) zugeführt und bei 190 °C getrocknet. Die Brüden des Trockners (14) werden zusammen mit der Mutterlauge aus der Filtration (15) in eine Destillation (16) geführt. Dort wird Chlorbenzol abdestilliert und in einen der Rührautoklaven (R) zurückgeführt. Aus dem Trockner werden stündlich 147,1 kg 1,5-Diaminoanthrachinon in einer Reinheit von 98,0% entnommen. Das entspricht einer Ausbeute von mehr als 97%.

Beispiel 3

Es wird wie im Beispiel 1 verfahren, jedoch anstelle von Chlorbenzol o-Xylol verwendet und die Sumpftemperatur der Ammoniakdestillation bei 208°C gehalten. Man erhält stündlich 146,9 kg 1,5-Diaminoanthrachinon in 98,1%iger Reinheit. Das entspricht einer Ausbeute von etwa 97%.

Beispiel 4 (Die Bezugszeichen beziehen sich auf die Fig. 1)

In einen von 3 parallel geschalteten Rührautoklaven (R), von denen in Fig. 1 nur einer abgebildet ist, bestehend aus rostfreiem Stahl, werden 340 kg Ammoniak und 1460 kg techn. Xylol eingepumpt und auf 140°C erhitzt, so dass sich ein Druck von 70 bar einstellt. In einem Rührbehälter (1) werden 220,5 kg 1,8-Dinitro-anthrachinon (Reinheit 98,5%) in 40 kg technisches Xylol suspendiert. Mit einer Kolbendosierpumpe (2) wird diese Suspension im Verlauf von 90 Minuten in den Rührautoklaven eindosiert. Die Temperatur im Autoklaven wird durch Wärmeabführung über einen Wärmetauscher (3), der über dem Füllstand im Rührautoklaven und in einem Raum angebracht ist, der in gutem Diffusionskontakt mit dem Gasraum im Rührautoklaven steht, bei 140 bis 150°C gehalten. Nach dem Zudosieren wird die Temperatur im Reaktor auf 180°C erhöht und bei dieser Temperatur 30 Minuten gehalten.

Nach einer Reaktionszeit von insgesamt 180 Minuten ist das 1,8-Dinitro-anthrachinon vollständig umgesetzt. Im Reaktor liegen 163,1 kg 1,8-Diamino-anthrachinon vor.

Die 3 parallel geschalteten Rührautoklaven werden zeitlich gegeneinander versetzt auf die gleiche Weise betrieben, und ein kontinuierlicher Strom des in Xylol suspendierten 1,8-Diaminoanthrachinons über Leitung 4 in eine Ammoniak-Destillation (5) so eingebracht, dass je Stunde etwa 81,6 kg reines 1,8-Diamino-anthrachinon aus einem der Rührautoklaven ausgetragen werden. Bei einer Sumpftemperatur von 205°C und 20 bar Druck werden Stickstoff und Ammoniak aus dem Reaktionsgemisch ausgetrieben. Ammoniak wird über einen Behälter (6) in kondensierter Form einem der Reaktoren (R) zugeführt. Stickstoff wird über eine Abgaswäsche mit Wasser in die Atmosphäre abgegeben.

Nach einer Verweilzeit von 6 Minuten im Sumpf der Ammoniak-Destillation (5) wird das Reaktionsgemisch auf Normaldruck in einen Behälter (7) entspannt. Dabei destilliert Wasser und Xylol ab, die in einem Trenngefäss (8) nach Phasen getrennt werden. Xylol wird über die Leitung (9) in einen der Rührautoklaven (R) zurückgeführt. Die wässrige Phase wird einer Destillation (10) zugeführt. Dort wird praktisch das ganze in der wässrigen Phase enthaltene Xylol zusammen mit Wasser über Kopf genommen und in den Trennbehälter (8) zurückgeführt. Als Sumpfprodukt verbleibt ein schadstofffreies Abwasser.

Die Produktsuspension aus dem Behälter (7) wird über einen Kühler (11) mit 40°C auf einen Filter (12) gebracht. Das Kristallisat wird einem Trockner (13) zugeführt und bei 190°C getrocknet. Die Brüden des Trockners (14) werden zusammen mit der Mutterlauge aus der Filtration (15) in eine Destillation (16) geführt. Dort wird Xylol abdestilliert und in einen der Rührautoklaven (R) zurückgeführt. Aus dem Trockner werden stündlich 80,2 kg 1,8-Diamino-anthrachinon in einer Reinheit von 97,3% entnommen.

**Patentansprüche**

1. Verfahren zur Herstellung von 1,5-Diamino-, 1,8-Diamino-, 1-Amino-6-nitro- und/oder 1-Amino-7-nitro-anthrachinon durch Umsetzung der entsprechenden Dinitroanthrachinone mit Ammoniak in einem organischen Reaktionsmedium bei Temperaturen im Bereich 130 bis 200°C, wobei Ammoniak bezogen auf 1 Mol Dinitroanthrachinon in Mengen von 5 bis 30 Mol eingesetzt wird, dadurch gekennzeichnet, dass man

a) als organisches Reaktionsmedium aliphatische, cycloaliphatische und/oder aromatische Kohlenwasserstoffe einsetzt, wobei die aromatischen Kohlenwasserstoffe alkylsubstituiert und/oder im Kern chloriert sein können;

b) Ammoniak und einen Teil des organischen Reaktionsmediums in einem Rührautoklaven vorlegt und auf eine Temperatur im Bereich von 130 bis 175°C erhitzt;

c) das Dinitroanthrachinon suspendiert im restlichen organischen Reaktionsmedium im Verlaufe von 30 bis 150 Minuten in die unter einem Druck im Bereich von 5 bis 120 bar stehende Vorlage eindosiert, wobei man oberhalb des Füllstandes des Rührautoklaven Wärme abzieht und so die Temperatur im organischen Reaktionsmedium im Bereich zwischen 140 und 175°C hält;

d) nach Beendigung der Zugabe von Dinitroanthrachinon die Temperatur im organischen Reaktionsmedium für 15 bis 150 Minuten um mindestens 10°C höher als in der Stufe c) und im Bereich von 170 bis 200°C und den Druck im Bereich von 30 bis 200 bar einstellt;

e) das Reaktionsgemisch aus dem Rührautoklaven in eine im Bereich von 10 bis 30 bar betriebene erste Destillationseinheit einspeist, in der Stickstoff und Ammoniak über Kopf genommen werden und in der die Verweilzeit im Abtriebsteil und im Sumpf im Bereich von 1 bis 30 Minuten liegt;

f) das Sumpfprodukt aus Stufe e) in eine im Bereich von 0,5 bis 5 bar betriebene zweite Destillationseinheit einspeist, in der Reaktionswasser und ein solcher Teil des organischen Reaktionsmediums über Kopf genommen wird, dass ein Sumpfprodukt mit einem Feststoffanteil im Bereich von 10 bis 40 Gew.-% anfällt;

g) das Sumpfprodukt aus Stufe f) entweder trocknet oder auf eine Temperatur unter 120°C abkühlt, die festen Anteile aus dem abgekühlten Sumpfprodukt abtrennt und die abgetrennten festen Bestandteile trocknet.

2. Verfahren nach Anspruch 1, dadurch gekennzeichnet, dass man in Stufe c) eine 10 bis 50% Feststoff enthaltende Suspension von Dinitroanthrachinon im organischen Reaktionsmedium und in Stufe b) so viel organisches Reaktionsmedium einsetzt, dass insgesamt 5 bis 15 Gewichtsteile des organischen Reaktionsmediums, bezogen auf 1 Gewichtsteil Dinitroanthrachinon, eingesetzt werden.

3. Verfahren nach Anspruch 1 und 2, dadurch gekennzeichnet, dass die Gesamtzeit für die Durchführung der Stufen c) und d) 90 bis 180 Minuten beträgt.

4. Verfahren nach Anspruch 1 bis 3, dadurch gekennzeichnet, dass in Stufe e) die Sumpftemperatur bei 200 °C oder darüber liegt.

5. Verfahren nach Anspruch 1 bis 4, dadurch gekennzeichnet, dass aus dem Kopfprodukt der Stufe e) Ammoniak durch Kondensation abgetrennt und in Stufe b) zurückgeführt wird.

6. Verfahren nach Anspruch 5, dadurch gekennzeichnet, dass der verbleibende Stickstoff einer Abgaswäsche mit Wasser zugeführt und dann in die Atmosphäre abgegeben wird.

7. Verfahren nach Anspruch 1 bis 6, dadurch gekennzeichnet, dass die in Stufe f) entweichenden Dämpfe kondensiert, in wässrige und eine organische Phase getrennt und die organische Phase in Stufe b) und/oder c) zurückgeführt wird.

8. Verfahren nach Anspruch 7, dadurch gekennzeichnet, dass die wässrige Phase einer dritten Destillationseinheit zugeführt wird, in der noch vorhandene Anteile des organischen Reaktionsmediums zusammen mit Wasser über Kopf abgenommen und in die Phasentrennung zurückgeführt werden.

9. Verfahren nach Anspruch 1 bis 8, dadurch gekennzeichnet, dass man die in Stufe g) bei der Trocknung entweichenden Brüden gegebenenfalls zusammen mit der Mutterlauge aus der Abtrennung der festen Anteile einer vierten Destillationseinheit zuführt, in der die in den Brüden und gegebenenfalls die in der Mutterlauge vorhandenen Anteile des organischen Reaktionsmediums über Kopf abgetrennt und in Stufe b) und/oder c) zurückgeführt werden.

10. Verfahren nach Anspruch 1 bis 9, dadurch gekennzeichnet, dass man das Verfahren kontinuierlich durchführt, wobei die Stufen b), c) und d) in mehreren Rührautoklaven durchgeführt werden, die zeitlich gegeneinander versetzt betrieben werden.

## Claims

1. Process for the preparation of 1,5-diamino-anthraquinone, 1,8-diamino-anthraquinone, 1-amino-6-nitro-anthraquinone and/or 1-amino-7-nitro-anthraquinone by reacting the corresponding dinitroanthraquinones with ammonia in an organic reaction medium at temperatures in the range from 130 to 200 °C, ammonia being employed in amounts of 5 to 30 mols relative to 1 mol of dinitroanthraquinone, characterised in that

a) aliphatic, cycloaliphatic and/or aromatic hydrocarbons are employed as the organic reaction medium, it being possible for the aromatic hydrocarbons to be alkyl-substituted and/or chlorinated in the nucleus;

b) ammonia and some of the organic reaction medium are initially introduced into a stirred autoclave and are heated to a temperature in the range from 130 to 175 °C;

c) the dinitroanthraquinone, suspended in the rest of the organic reaction medium, is metered into the initial mixture, which is under a pressure in the range from 5 to 120 bars, in the course of 30 to 150 minutes, heat being removed above the level to which the stirred autoclave is filled and the temperature in the organic reaction medium thus being kept in the range between 140 and 175 °C;

d) when the addition of dinitroanthraquinone is finished, the temperature in the organic reaction medium is adjusted to at least 10 °C higher than that in stage c), and in the range from 170 to 200 °C, for 15 to 150 minutes and the pressure is adjusted to a pressure in the range from 30 to 200 bars;

e) the reaction mixture is fed from the stirred autoclave into a first distillation unit which operates in the range from 10 to 30 bars and in which nitrogen and ammonia are taken off over the top and in which the residence time in the stripping section and at the bottom is in the range from 1 to 30 minutes;

f) the bottom product from stage e) is fed into a second distillation unit, which operates in the range from 0.5 to 5 bars and in which the water of reaction and an amount of the organic reaction medium such that a bottom product with a solids constituent in the range from 10 to 40% by weight is obtained are taken off over the top; and

g) the bottom product from stage f) is either dried or cooled to a temperature below 120 °C, the solid constituents of the cooled bottom product are separated off and the solid constituents separated off are dried.

2. Process according to Claim 1, characterised in that a suspension, containing 10 to 50% of solid, of dinitroanthraquinone in the organic reaction medium is added in stage c) and an amount of organic reaction medium such that a total of 5 to 15 parts by weight of the organic reaction medium, relative to 1 part by weight of dinitroanthraquinone, is employed, is added in stage b).

3. Process according to Claim 1 and 2, characterised in that the total time for carrying out stages c) and d) is 90 to 180 minutes.

4. Process according to Claim 1 to 3, characterised in that the bottom temperature in stage e) is 200 °C or more.

5. Process according to Claim 1 to 4, characterised in that ammonia is separated off, by condensation, from the top product from stage e) and recycled into stage b).

6. Process according to Claim 5, characterised in that the nitrogen which remains is passed to an off-gas wash with water and then released into the atmosphere.

7. Process according to Claim 1 to 6, characterised in that the vapours escaping in stage 5) are condensed and separated into an aqueous phase and an organic phase and the organic phase is recycled into stage b) and/or c).

8. Process according to Claim 7, characterised in that the aqueous phase is fed to a third distillation unit in which constituents of the organic reaction medium still present are taken off over the top, together with water, and recycled into the phase separation.

9. Process according to Claim 1 to 8, characterised in that the vapours escaping during drying in stage g) are fed, if appropriate together with the mother liquor obtained whilst separating off the solid constituents, to a fourth distillation unit in which the constituents of the organic reaction medium present in the vapours and, if appropriate, those present in the mother liquor are separated off over the top and recycled into stage b) and/or c).

10. Process according to Claim 1 to 9, characterised in that the process is carried out continuously, stages b), c) and d) being carried out in several stirred autoclaves, which are operated at staggered times.

**Revendications**

1. Procédé de production de 1,5-diamino-, 1,8-diamino-, 1-amino-6-nitro- et/ou 1-amino-7-nitro-anthraquinones par réaction des dinitroanthraquinones correspondantes avec l'ammoniac dans un milileu réactionnel organique à des températures comprises dans la plage de 130 à 200 °C, l'ammoniac étant utilisé en quantités de 5 à 30 moles par mole de dinitro-anthraquinone, caractérisé en ce que:

a) on utilise comme milieu réactionnel organique des hydrocarbures aliphatiques, cycloaliphatiques et/ou aromatiques, les hydrocarbures aromatiques pouvant porter des substituants alkyle et/ou pouvant être chlorés dans le noyau;

b) on introduit préalablement de l'ammoniac et une partie du milieu réactionnel organique dans un autoclave équipé d'un agitateur et on les chauffe à une température comprise dans la plage de 130 à 175 °C;

c) on injecte la dinitro-anthraquinone en suspension dans le milieu réactionnel organique restant, en une période de 30 à 150 minutes, dans le mélange préalable sous pression comprise dans la plage de 5 à 120 bars (0,5 à 12 MPa), en dissipant de la chaleur au-dessus du niveau de l'autoclave à agitateur et en maintenant ainsi la température du milieu réactionnel organique dans la plage de 140 à 175 °C;

d) on règle la température du milieu réactionnel organique pendant 15 à 150 minutes à une valeur plus haute d'au moins 10 °C que dans l'étape (c) et dans la plage de 170 à 200 °C et la pression dans la plage de 30 à 200 bars (3 à 20 MPa) après la fin de l'addition de la dinitro-anthraquinone;

e) on injecte le mélange réactionnel de l'autoclave à agitateur dans une première unité de distillation fonctionnant dans la plage de 10 à 30 bars (1 à 3 MPa), dans laquelle de l'azote et de l'ammoniac sont éliminés en tête et la durée de séjour dans la partie de séparation et dans le puisard se situe dans la plage de 1 à 30 minutes;

f) le résidu de l'étape (e) est injecté dans une seconde unité de distillation fonctionnant dans la plage de 0,5 à 5 bars (0,05 à 0,5 MPa) en tête de laquelle sont éliminées l'eau de réaction et une partie du milieu réactionnel organique telle que l'on obtienne un résidu ayant une teneur en matières solides de 10 à 40% en poids;

g) on sèche le résidu de l'étape (f) ou on le refroidit à une température inférieure à 120 °C, on sépare les composants solides du résidu refroidi et on sèche les composants solides séparés.

2. Procédé suivant la revendication 1, caractérisé en ce que dans l'étape (c), on utilise une suspension de dinitro-anthraquinone à 10–50% de matière solide dans le milieu réactionnel organique et dans l'étape (b), on utilise une quantité suffisante de milieu réactionnel organique pour qu'il y ait au total 5 à 15 parties en poids de milieu réactionnel organique pour 1 partie en poids de dinitro-anthraquinone.

3. Procédé suivant l'une des revendications 1 et 2, caractérisé en ce que la durée totale de conduite des étapes (c) et (d) va de 90 à 180 minutes.

4. Procédé suivant l'une des revendications 1 à 3, caractérisé en ce que dans l'étape (e), la température du puisard est égale ou supérieure à 200 °C.

5. Procédé suivant l'une des revendications 1 à 4, caractérisé en ce que de l'ammoniac est séparé du produit de tête de l'étape (e) par condensation et recyclé dans l'étape (b).

6. Procédé suivant la revendication 5, caractérisé en ce que l'azote restant est soumis à un lavage à l'eau des gaz résiduaires, puis déchargé dans l'atmosphère.

7. Procédé suivant l'une des revendications 1 à 6, caractérisé en ce que les vapeurs qui s'échappent dans l'étape (f) sont condensées, séparées en une phase aqueuse et une phase organique et la phase organique est recyclée dans l'étape (b) et/ou dans l'étape (c).

8. Procédé suivant la revendication 7, caractérisé en ce que la phase aqueuse est introduite dans une troisième unité de distillation dans laquelle les composants encore présents du milieu réactionnel organique sont chassés en tête en même temps que l'eau et ramenés à la séparation des phases.

9. Procédé suivant l'une des revendications 1 à 8, caractérisé en ce que les vapeurs chaudes qui s'échappent lors du séchage dans l'étape (g) sont amenées, le cas échéant en même temps que la liqueur-mère de séparation des composants solides, à une quatrième unité de distillation dans laquelle les composants du milieu réactionnel organique présents dans les vapeurs chaudes et, le cas échéant, présents dans la liqueur-mère sont séparés en tête et recyclés dans l'étape (b) et/ou dans l'étape (c).

10. Procédé suivant l'une des revendications 1 à 9, caractérisé en ce qu'il est mis en œuvre en continu, les étapes (b), (c) et (d) étant conduites dans plusieurs autoclaves pourvus d'un agitateur, qui sont actionnés avec un décalage dans le temps les uns par rapport aux autres.